# EUROPEAN PATENT APPLICATION

(11) **EP 1 961 381 A1**
(43) Date of publication of application: **27.08.2008**
(21) Application number: 06833761.7
(22) Date of filing: 30.11.2006
(51) Int. Cl.: A61B 5/151, A61B 5/15, A61B 5/157

(54) **SENSOR/LANCET INTEGRATED DEVICE AND METHOD OF COLLECTING BODY FLUID USING THE SAME**

(30) Priority: 01.12.2005 JP 2005347521
(71) Applicant: Arkray, Inc., Kyoto-shi, Kyoto 601-8045 (JP)
(72) Inventor: DOI, Shigeru, Kyoto 601-8045 (JP)
(74) Representative: HOFFMANN EITLE
(86) International application number: PCT/JP2006/323958
(87) International publication number: WO 2007/063948

(57) **Abstract**

The present invention relates to a sensor/lancet integrated device 1 including a lancet including a needle part 14 for puncturing skin, and a sensor including a reagent part 17. The lancet is integrally molded with the needle part 14. The device 1 preferably further includes a capillary 15 for supplying to the reagent part 17. Preferably, the needle part 14 is formed to a hollow form, and has the interior communicated to an introduction port. The needle part 14 may be formed projecting from a location different from the introduction port.

## Description

### TECHNICAL FIELD

The present invention relates to a sensor/lancet integrated device having both a function of a sensor and a function of a lancet. The present invention also relates to a blood collecting method using the device.

### BACKGROUND ART

When measuring a blood glucose level, a method of incising the skin to bleed blood from the skin, and supplying the blood to an analyzing tool such as a biosensor is adopted. The incision of the skin is performed by attaching a lancet to a lancing device, and puncturing a needle part of the lancet to the skin.

The lancing device used only for the purpose of incising the skin is known, but that configured so that incision of the skin and measurement of the blood glucose level are simultaneously carried out using a device in which a biosensor and a needle part are integrated is also known (refer to, e.g., Patent Documents 1, 2).

Patent Document 1: Japanese Unexamined Patent Publication No. 2000-254112
Patent Document 2: U.S. Patent No. 4627445

### DISCLOSURE OF THE INVENTION

### PROBLEMS TO BE SOLVED BY THE INVENTION

However, a conventional device has problems in that the number of components is large and the structure is complex, and thus workability in manufacturing is poor and the manufacturing cost is high.

It is an object of the present invention to provide a device with a function of a sensor and a function of a lancet having a simple structure of small number of components, and being manufactured easily and at low manufacturing cost.

### MEANS FOR SOLVING THE PROBLEMS

In a first aspect of the present invention, there is provided a sensor/lancet integrated device including a lancet including a needle part for puncturing skin, and a sensor including a reagent part; wherein the lancet is integrally molded with the needle part.

The device of the present invention further includes a capillary for supplying body fluid to the reagent part etc. In this case, the needle part is projected from a location different from an introduction port of the body fluid in the capillary. The needle part is formed hollow, and may be fixed to communicate the interior of the needle part to the capillary.

The lancet may be formed including a groove for defining the capillary.

In a second aspect of the present invention, there is provided a sensor/lancet integrated device including a lancet including a needle part for puncturing skin, and a sensor including an introduction port for introducing body fluid; wherein the needle part is formed projecting from a location different from the introduction port. For instance, the needle part is integrally molded to a component of the sensor.

The device of the present invention may also include a cover to be joined to the sensor, and including a groove for defining a capillary for introducing the body fluid.

In a third aspect of the present invention, there is provided a sensor/lancet integrated device including a lancet including a needle part for puncturing skin, and a sensor including a reagent part; wherein the sensor has a mode in which a cover is joined with respect to a substrate; and the needle part is fixed between the substrate and the cover.

The sensor has a mode in which a cover is joined with respect to a substrate etc. In this case, the lancet includes a joint for attaching to the sensor. In this case, the lancet is fixed to the sensor so as to sandwich the substrate with the joint. The lancet may be fixed to the sensor so as to further sandwich the cover with the joint.

In a fourth aspect of the present invention, there is provided a sensor/lancet integrated device including a lancet including a needle part for puncturing skin, and a sensor including a reagent part; wherein the lancet includes a joint for fixing to the sensor; and the needle part is integrated to the joint.

The sensor further includes an electrode etc. That is, the present invention can be applied to a device configured to analyze a specific component in the body fluid such as blood using an electrochemical method.

The device of the present invention may be configured such that the sensor and the lancet are formed as separate bodies; and the sensor is held in an internal space formed in the lancet to be integrated to each other.

The device of the present invention is formed with the reagent part including a color producing agent in an internal space formed in the lancet; and at least one part of the lancet is formed transparent to measure a color of the color producing agent. That is, the present invention can be applied to a device configured to analyze a specific component in the body fluid such as blood using an optical method.

In a fifth aspect of the present invention, there is provided a body fluid collecting method using a sensor/lancet integrated device including a lancet integrally molded with a needle part formed to a hollow form, and a sensor with a reagent part; the method including a first step of moving the needle part to a position where a tip of the needle part contacts the skin, and obtaining the position where the tip of the needle part contacts the skin; a second step of puncturing the needle part to the skin and incising the skin; a third step of removing the needle part from the skin; and a fourth step of contacting the tip of the needle part to the body fluid bled from the skin based on a result in the first step.

For instance, the fourth step is performed by again moving the needle part after once being moved to a position where the tip of the needle part is completely away from the skin, and contacting the needle part to the body fluid bled from the skin. The fourth step is performed by stopping the movement of the needle part immediately after removing the needle part from the skin.

At least one of the second step or the third step is preferably performed with a negative pressure applied to the skin.

In a sixth aspect of the present invention, there is provided a body fluid collecting method using a sensor/lancet integrated device including a lancet integrally molded with a needle part formed to a hollow form, and a sensor including a reagent part; the method including a first step of puncturing the needle part to the skin and incising the skin; and a second step of aspirating the body fluid to an interior of the needle part by a capillary force generated in the interior of the needle part with the needle part punctured in the skin.

In a seventh aspect of the present invention, there is provided a body fluid collecting method using a sensor/lancet integrated device including a sensor with an introduction port for introducing body fluid, and a lancet with a needle part formed projecting from a location different from the introduction port; the method including a first step of puncturing the needle part to the skin and incising the skin; a second step of removing the needle part from the skin; a third step of rotating the sensor/lancet integrated device; and a fourth step of contacting the introduction port to the body fluid bled from the skin.

Preferably, the body fluid collecting method according to the sixth aspect of the present invention further includes a fifth step, performed before the first step, of moving the needle part to a position where a tip of the needle part contacts the skin, and obtaining the position where the tip of the needle part contacts the skin.

### BRIEF DESCRIPTION OF THE DRAWINGS

Fig. 1 is a plan view showing a sensor/lancet integrated device according to a first embodiment of the present invention.
Fig. 2 is a cross-sectional view taken along line II-II of Fig. 1.
Fig. 3 is a cross-sectional view for describing a lancing device using the device shown in Fig. 1.
Fig. 4 is a cross-sectional view showing, in an enlarged manner, the main part of the lancing device shown in Fig. 3.
Fig. 5 is a cross-sectional view for describing a blood collecting method using the device shown in Fig. 1 and the lancing device shown in Fig. 3.
Fig. 6 is a cross-sectional view showing main parts for describing the blood collecting method using the device shown in Fig. 1 and the lancing device shown in Fig. 3.
Fig. 7 is a cross-sectional view showing the main parts for describing another example of the blood collecting method using the device shown in Fig. 1 and the lancing device shown in Fig. 3.
Fig. 8 is a plan view showing a sensor/lancet integrated device according to a second embodiment of the present invention.
Fig. 9 is a cross-sectional view taken along line IX-IX of Fig. 8.
Fig. 10 is an exploded perspective view of the device shown in Fig. 8.
Fig. 11 is an overall perspective view showing a sensor/lancet integrated device according to a third embodiment of the present invention.
Fig. 12 is an exploded perspective view of the device shown in Fig. 11.
Fig. 13 is a cross-sectional view taken along line XIII-XIII of Fig. 11.
Fig. 14 is an overall perspective view showing a sensor/lancet integrated device according to a fourth embodiment of the present invention.
Fig. 15 is an exploded perspective view of the device shown in Fig. 14.
Fig. 16 is a cross-sectional view taken along line XVI-XVI of Fig. 14.
Fig. 17 is a cross-sectional view showing another example of a lancet in the device shown in Fig. 14.
Fig. 18 is an overall perspective view showing a sensor/lancet integrated device according to a fifth embodiment of the present invention.
Fig. 19 is an exploded perspective view of the device shown in Fig. 18.
Fig. 20 is a cross-sectional view taken along line XX-XX of Fig. 18.
Fig. 21 is a cross-sectional view showing another example of a needle part in the device shown in Fig. 18.
Fig. 22 is an overall perspective view showing a sensor/lancet integrated device according to a sixth embodiment of the present invention.
Fig. 23 is an exploded perspective view of the device shown in Fig. 22.
Fig. 24 is a cross-sectional view taken along line XXIV-XXIV of Fig. 22.
Fig. 25 is a plan view showing a sensor/lancet integrated device according to a seventh embodiment of the present invention.
Fig. 26 is a plan view showing a sensor/lancet integrated device according to an eighth embodiment of the present invention.
Fig. 27 is a cross-sectional view taken along line XXVII-XXVII of Fig. 26.
Fig. 28 is an overall perspective view showing a sensor/lancet integrated device according to a ninth embodiment of the present invention.
Fig. 29 is an exploded perspective view of the device shown in Fig. 28.
Fig. 30 is a cross-sectional view taken along line XXX-XXX of Fig. 28.
Fig. 31 is a cross-sectional view for describing a lancing device using the device shown in Fig. 28.
Fig. 32 is a partially cut side view showing the main parts of the lancing device shown in Fig. 31.
Fig. 33 is a front view showing the main parts for describing a rotation mechanism of the lancing device shown in Fig. 31.
Fig. 34 is a cross-sectional view showing the main parts for describing a blood collecting method using the device shown in Fig. 28 and the lancing device shown in Fig. 31.
Fig. 35 is a cross-sectional view showing the main parts for describing a blood collecting method using the device shown in Fig. 28 and the lancing device shown in Fig. 31.
Fig. 36 is a cross-sectional view showing the main parts for describing a blood collecting method using the device shown in Fig. 28 and the lancing device shown in Fig. 31.
Fig. 37 is an overall perspective view showing a sensor/lancet integrated device according to a tenth embodiment of the present invention.
Fig. 38 is an exploded perspective view of the device shown in Fig. 37.
Fig. 39 is a cross-sectional view taken along line XXXIX-XXXIX of Fig.37.
Fig. 40 is a plan view showing a sensor/lancet integrated device according to an eleventh embodiment of the present invention.

### BEST MODE FOR CARRYING OUT THE INVENTION

First to eleventh embodiments of the present invention will be hereinafter described with reference to the drawings.

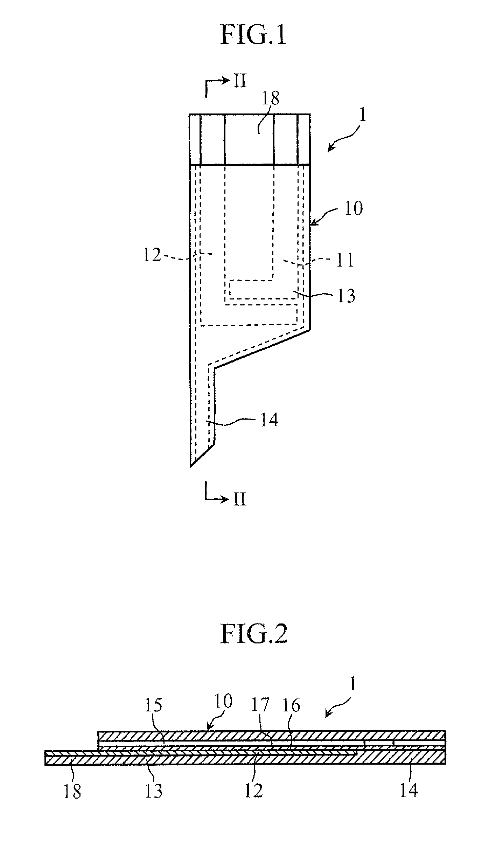

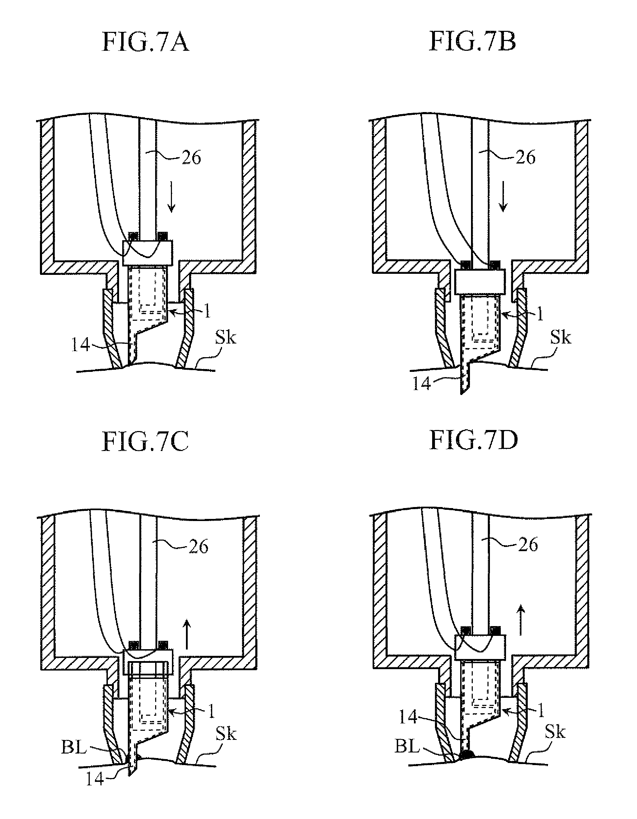

First, a first embodiment of the present invention will be described with reference to Fig. 1 and Fig. 7.

A sensor/lancet integrated device 1 shown in Fig. 1 and Fig. 2 have functions of the sensor and the lancet, and includes a resin molded body 10 and a pair of electrodes 11, 12.

The entire resin molded body 10 is integrally formed by resin molding, and is arranged with a body part 13 and a needle part 14.

The body part 13 has an internal space 15 for acting a capillary force, where a reagent part 16 is formed in the internal space 15. The reagent part 16 dissolves by the blood supplied to the body part 13, and contains oxidation-reduction enzyme, electron transfer substance, and the like. Such reagent part 16 can be formed by holding material liquid containing oxidation-reduction enzyme and electron transfer substance in the internal space 15 of the body part 13, and drying the material liquid by air-blow drying or freeze-drying.

The needle part 14 is provided to puncture the skin when incising the skin, and to extract blood bled from the skin. The needle part 14 is formed to a hollow form, and is configured so that capillary force generates in the interior of the needle part 14. The interior of the needle part 14 is communicated to the internal space 15 of the body part 13, where the blood is collected from the needle part 14 and such blood is supplied to the internal space 15 of the body part 13 by the capillary force generated in the needle part 14 and the body part 13 when the tip of the needle part 14 is contacted to the blood bled from the skin. When the blood is supplied to the internal space 15, the reagent part 16 dissolves by the blood, and a liquid phase reaction system develops in the internal space 15.

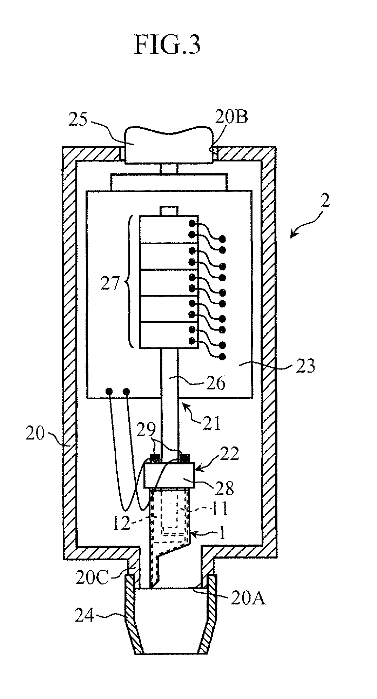

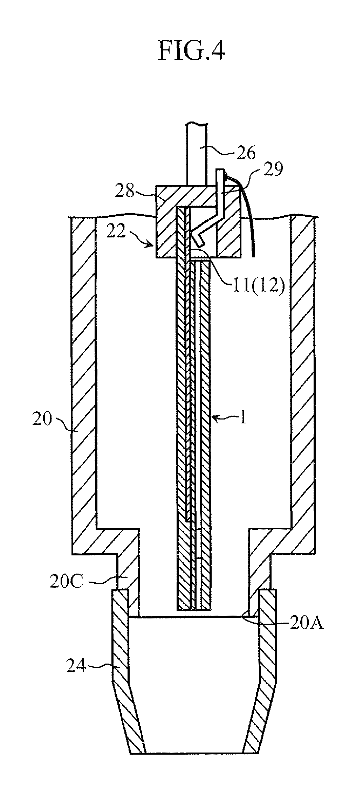

A pair of electrodes 11, 12 applies voltage to the liquid phase reaction system developed in the internal space 15 of the body part 13, and measures the response current thereof. Each electrode 11, 12 have a surface 17 facing the internal space 15 of the body part 13, and are exposed to the outside at a projection 18 of the body part 13. Each electrode 11, 12 contacts the reagent part 16 in the internal space 15, and can be contacted to a connector 22 (see Fig. 3 and Fig. 4) of a lancing device 2 to be hereinafter described at the projection 18. Such electrodes 11, 12 are formed by insert molding a metal plate and the like to the resin molded body 10.

As shown in Fig. 3 and Fig. 4, the sensor/lancet integrated device 1 is used by being attached to the lancing device 2. The lancing device 2 includes a movable mechanism 21, the connector 22, and a control plate 23 inside a housing 20.

The housing 20 accommodates various elements and defines the outer shape of the device, and has openings 20A, 20B. The opening 20A is the portion covered by a cap 24. The cap 24 is detachably fixed at a flange 20C defining the opening 20A. The opening 20B allows movement of an operation button 25. The operation button 25 generates a signal to start the measurement.

The movement mechanism 21 is provided to move the device 1, and includes a rod 26 and an actuator 27. The rod 26 is connected to the connector 22, and is formed to a circular cross-section by a conductor such as metal. The actuator 27 is provided to reciprocate the rod 26 in the up and down direction. The actuator 27 is configured so that the rod 26 is moved by a voice coil motor or a solenoid. The actuator 27 is conduction connected to the control plate 23. The movement mechanism 21 may have a configuration of changing the rotary motion of the motor to a linear motion of the rod 26.

The connector 22 applies voltage between the electrodes 11, 12 of the device 1, and measures the response current at the time. The connector 22 also has a role of holding the device 1. The connector 22 includes a holder part 28 and a pair of terminals 29. The holder part 28 is coupled to the rod 26, and is movable in the up and down direction with the rod 26. The terminal 29 contacts the electrodes 11, 12 of the device 1 at the end, and fixed to the holder part 28. The terminal 29 is conduction connected to the control plate 23, and is configured as a plate spring. That is, with the terminal 29 contacted to the electrodes 11, 12 of the device 1, it is acted with a pushing force by the electrode 11, 12, and furthermore, the device 1 by the elastic force of the terminal 29. As a result, the device 1 is held by the connector 22.

The control plate 23 controls the up and down movement of the rod 26, the application state of the voltage on the terminal 29, the measurement of the response current value through the terminal 29, and the like. For instance, a switch (not shown) is turned ON when the operation button 25 is pushed down, whereby the measurement start signal is generated and such signal is provided to the control plate 23. The rod 26 then performs a predetermined up and down movement, and the voltage is applied between the electrodes 11, 12 of the device 1 via the terminal 29 by the control plate 23. The control plate 23 uses the terminal 29 to obtain the response current value, and calculate the concentration of a specific component such as glucose in the body fluid such as blood.

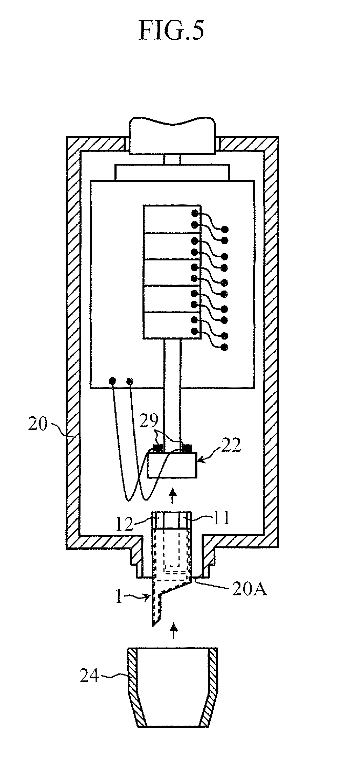

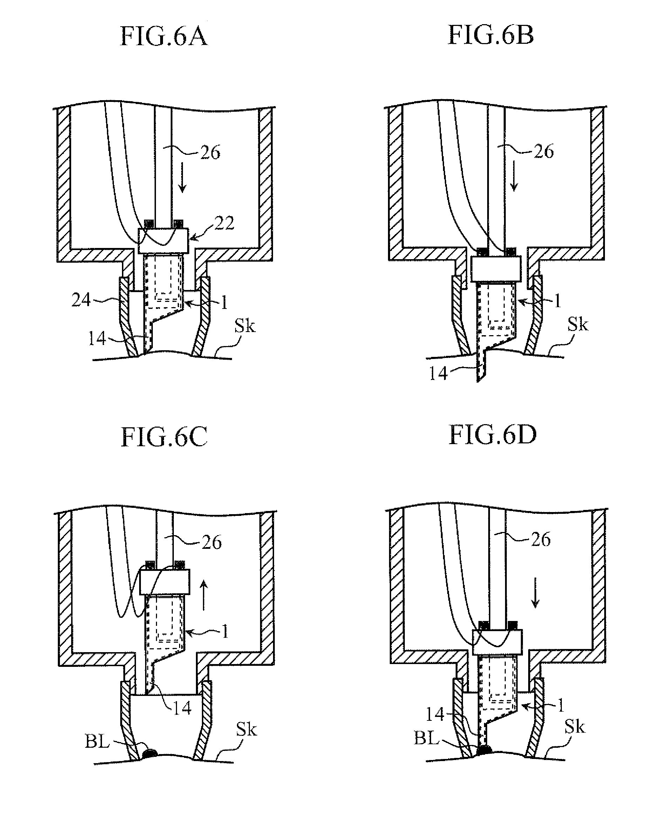

The measuring operation using the device 1 and the lancing device 2 will be described with reference to Fig. 5 and Fig. 6. In the following description, Fig. 1 to Fig. 3 are referenced as needed along with the instructed figure.

As shown in Fig. 5, first the cap 24 is detached from the housing 20 to open the opening 20A. The device 1 is inserted to the connector 22 in this state. When the device 1 is inserted to the connector 22, the terminal 29 of the connector 22 contacts the electrodes 11, 12 of the device 1, and the terminal 29 acts the pushing force on the device 1, whereby the device 1 is held by the connector 22 (see Fig. 4). After attachment of the device 1, the cap 24 is attached to the flange 20C, and the opening 20A is covered with the cap 24 to obtain the state shown in Fig. 3.

Then, as shown in Fig. 6A, the operation button 25 is pushed down with the distal end of the cap 24 contacting the skin Sk. The measurement start signal is thereby generated, and such signal is provided to the control plate 23. When detecting such signal, the control plate 23 controls the actuator 27 and moves the rod 26 downward. The connector 22 and the device 1 are then moved downward with the rod 26. When the tip of the needle part 14 of the device 1 contacts the skin Sk, the control plate 23 obtains the movement distance of the rod 26 to the relevant point. That is, the control plate 23 obtains the distance to the skin Sk from the state shown in Fig. 3. Whether the tip of the needle part 14 in the device 1 has contacted the skin is determined by detecting the time point at which the load acting on the rod 26 becomes large by a pressure sensor, and the like.

As shown in Fig. 6B, the control plate 23 further moves the rod 26 and furthermore the device 1 downward to puncture the skin Sk with the needle part 14 of the device 1.

As shown in Fig. 6C, the control plate 23 controls the actuator 27 after puncturing the skin Sk with the needle part 14, and moves the rod 26 and furthermore the device 1 upward to remove the needle part 14 from the skin Sk. Here, the puncture site of the skin Sk is incised by the needle part 14, and thus blood BL is bleeding from the puncture site.

As shown in Fig. 6D, the control plate 23 controls the actuator 27 to move the rod 26 and furthermore the device 1 downward to contact the needle part 14 of the device 1 to the skin Sk. The needle part 14 of the device 1 is appropriately contacted to the skin Sk since the distance to the skin Sk is known in the operation shown in Fig. 6A. Since the blood BL is bleeding at the puncture site from the skin Sk, the tip of the needle part 14 is contacted to the blood BL. The capillary force is acting in the needle part 14 as described above, and thus the blood BL bled from the skin Sk is introduced into the internal space 15 of device 1 through the needle part 14. Since the capillary force is also acting in the internal space 15, the internal space 15 is filled with blood BL. Here, the reagent part 16 is dissolved, and a reaction system containing reagent and blood is formed in the internal space 15.

When detecting the signal generated by the pushing of the operation button 25, the control plate 23 applies voltage between the terminals 29. The voltage is thereby applied to the reaction system of the internal space 15. The response current is then measured by the terminal 29. The control plate 23 also calculates the concentration of a specific component in the blood based on the response current measured by the terminal 29. Such calculation can be performed based on a known method such as an analytical curve showing a relationship between response current value and concentration.

Other examples of the blood collecting operation using the device 1 and the lancing device 2 will be described with reference to Fig. 7A to Fig. 7D. The following description references Fig. 2 and Fig. 3 as necessary along with the instructed figures.

First, as shown in Fig. 7A and Fig. 7B, the actuator 27 is controlled by the control plate 23 to move the rod 26 downward, and the needle part 14 of the device 1 is inserted into the skin Sk. In this process, when the tip of the needle part 14 contacts the skin Sk (state shown in Fig. 7A), the movement distance of the rod 26 to this point is obtained.

As shown in Fig. 7C and Fig. 7D, the rod 26 is moved upward, and the needle part 14 of the device 1 is gradually removed from the skin Sk. In such a way, the blood BL bleeds from the puncture site of the skin Sk incised by the needle part 14. The upward movement of the device 1 is stopped with the tip of the needle part 14 contacting the puncture site of the skin Sk. The tip of the needle part 14 is contacted to the blood BL and the blood BL is introduced into the interior of the device 1.

As a blood collecting method, a method of introducing blood into the interior of the needle part 14 by the capillary force generated in the interior of the needle part 14 with the needle part 14 inserted in the skin Sk can be adopted, as shown in Fig. 7B.

In the sensor/lancet integrated device 1, the needle part 14 is integrally formed by resin molding. The electrodes 11, 12 can be incorporated in the resin molded body 10 by insert molding. The sensor/lancet integrated device 1 thus has small number of components and a simple configuration, and can be easily manufactured using a resin mold. As a result, the sensor/lancet integrated device 1 can provide a function of a sensor and a function of a lancet to one device at low cost. Since the device 1 can be manufactured at low cost, the blood collecting cost can be reduced in the blood collecting method using the device 1.

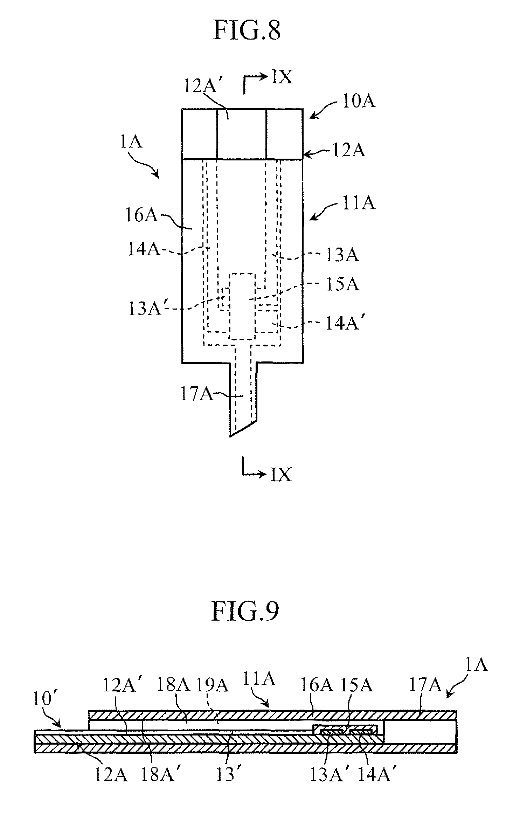

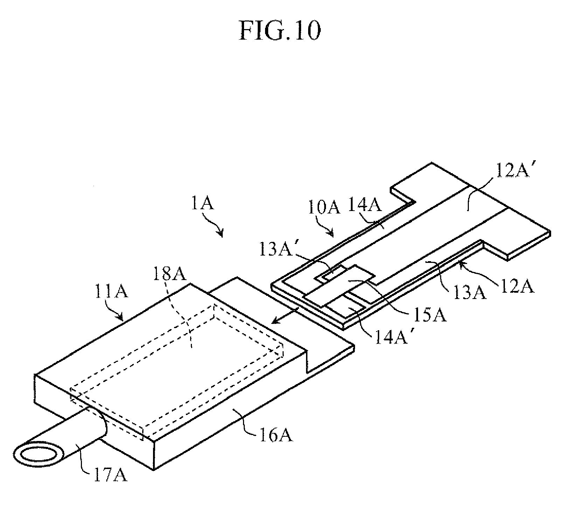

A second embodiment of the present invention will be described with reference to Fig. 8 to Fig. 10.

A sensor/lancet integrated device 1A shown in Fig. 8 and Fig. 9 includes a sensor 10A and a lancet 11A formed as separate bodies.

The sensor 10A has a pair of electrodes 13A, 14A and a reagent part 15A formed on an upper surface 12A' of a substrate 12A. The pair of electrodes 13A, 14A function similar to the pair of electrodes 11, 12 (see Fig. 1 and Fig. 2) in the previously described sensor/lancet integrated device 1, and are formed through screen printing and the like using carbon paste etc. The reagent part 15A is formed bridging across ends 13A' and 14A' of the electrodes 13A and 14A.

The entire lancet 11A is integrally formed by resin molding, and is arranged with a body part 16A and a needle part 17A.

The body part 16A has an internal space 18A for holding the sensor 10A. The internal space 18A is formed with the height dimension greater than the thickness dimension of the sensor 10A, where a gap 19A is formed between the sensor 10A and an upper surface 18A' defining the internal space 18A with the sensor 10A accommodated in the internal space 18A. The gap 19A functions as a capillary for acting the capillary force.

The needle part 17A functions similar to the needle part 14 (see Fig. 1 and Fig. 2) in the previously described sensor/lancet integrated device 1, and is integrally formed with the body part 16A to a hollow form.

The sensor/lancet integrated device 1A is used by being attached to the lancing device 2 described with reference to Fig. 3 and Fig. 4, similar to the previously described sensor/lancet integrated device 1 (see Fig. 1 and Fig. 2). In other words, in the sensor/lancet integrated device 1A, the device 1A is moved by the movement mechanism 21 (see Fig. 3 and Fig. 4) in the lancing device 2 to puncture the needle part 17A to the skin and collect blood from the skin. As a method of collecting blood from the skin, the method described with reference to Fig. 6 or the method described with reference to Fig. 7 can be applied. The specific component in the blood can be analyzed by applying voltage between the electrodes 13A, 14A by the connector 22 (see Fig. 3 and Fig. 4), and measuring the response current value.

The sensor/lancet integrated device 1A has both the function of the sensor and the function of the lancet by integrally forming the needle part 17A to the lancet 11A by resin molding, and holding the sensor 10A at the lancet 11A. The sensor/lancet integrated device 1A thus has small number of components and a simple configuration, and can be manufactured easily and effectively in terms of cost.

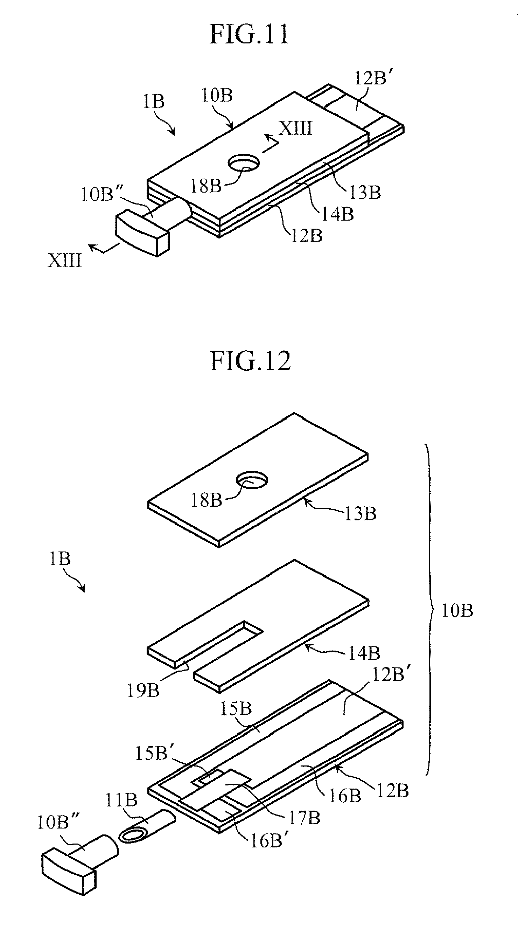

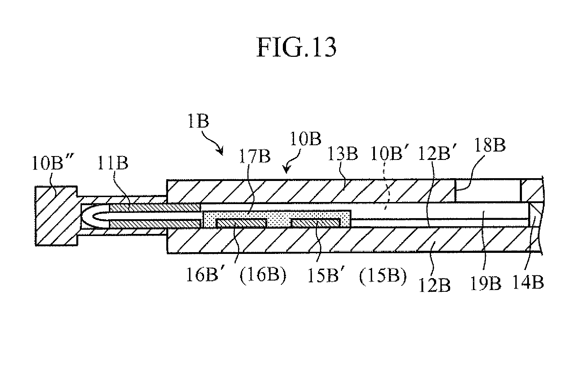

A third embodiment of the present invention will be described with reference to Fig. 11 to Fig. 13.

A sensor/lancet integrated device 1B shown in Fig. 11 to Fig. 13 includes a sensor 10B and a needle part 11B.

The sensor 10B has a spacer 14B interposed between a substrate 12B and a cover 13B, and is entirely formed to a plate shape.

The substrate 12B has a pair of electrodes 15B, 16B and a reagent part 17B formed on an upper surface 12B'. The pair of electrodes 15B, 16B function similar to the pair of electrodes 11, 12 (see Fig. 1 and Fig. 2) in the previously described sensor/lancet integrated device 1, and is formed through screen printing and the like using carbon paste etc. The reagent part 17B is formed bridging across ends 15B' and 16B' of the electrodes 15B, 16B.

A cover 13B includes a pass-through hole 18B and is arranged to cover the substrate 12B.

The spacer 14B defines a distance between the substrate 12B and the cover 13B, and includes a slit 19B. The slit 19B defines a capillary 10B' in the sensor 10B. The capillary 10B' is communicated to the pass-through hole 18B of the cover 13B. That is, the gas inside the capillary 10B' can be discharged to the outside through the pass-through hole 18B.

The needle part 11B functions similar to the needle part 14 (see Fig. 1 and Fig. 2) of the previously described sensor/lancet integrated device 1, and is formed to a hollow form. The needle part 11B is made from resin or metal, and is configured to act the capillary force. The needle part 11B is fixed between the substrate 12B and the cover 13B at the entrance of the capillary 10B'. The fixation of the needle part 11B may be carried out by sandwiching the needle part 11B between the substrate 12B and the cover 13B, or may be carried out using adhesive and the like. The needle part 11B is covered by a cap 10B".

The cap 10B" is removed when using the device 1B. In other words, the needle part 11B is prevented from being contaminated by covering the needle part 11B with the cap 10B" when the device 1B is not in use. The sterile state of the needle part 11B can be appropriately maintained by the cap 10B" by performing a sterilization process with the needle part 11B covered with the cap 10B". The cap 10B" can also be attached to the needle part 11B after using the device 1B. In this case, the user will not be injured by the needle part 11B, or the blood attached to the needle part 11B will not contaminate the user, and thus is hygienic.

The sensor/lancet integrated device 1B is used by being attached to the lancing device 2 described with reference to Fig. 3 and Fig. 4, similar to the previously described sensor/lancet integrated device 1 (see Fig. 1 and Fig. 2). In other words, in the sensor/lancet integrated device 1B, the device 1B is moved by the movement mechanism 21 (see Fig. 3 and Fig. 4) in the lancing device 2 to puncture the needle part 11B to the skin and collect blood from the skin. As a method of collecting blood from the skin, the method described with reference to Fig. 6 or the method described with reference to Fig. 7 can be applied. The specific component in the blood can be analyzed by applying voltage between the electrodes 15B, 16B by the connector 22 (see Fig. 3 and Fig. 4), and measuring the response current value.

The sensor/lancet integrated device 1B has both the function of the sensor and the function of the lancet although the needle part 11B is formed as a separate body through resin molding by fixing the needle part 11B at the entrance of the capillary 10B'. The sensor/lancet integrated device 1B thus has small number of components and a simple configuration, and can be manufactured easily and effectively in terms of cost.

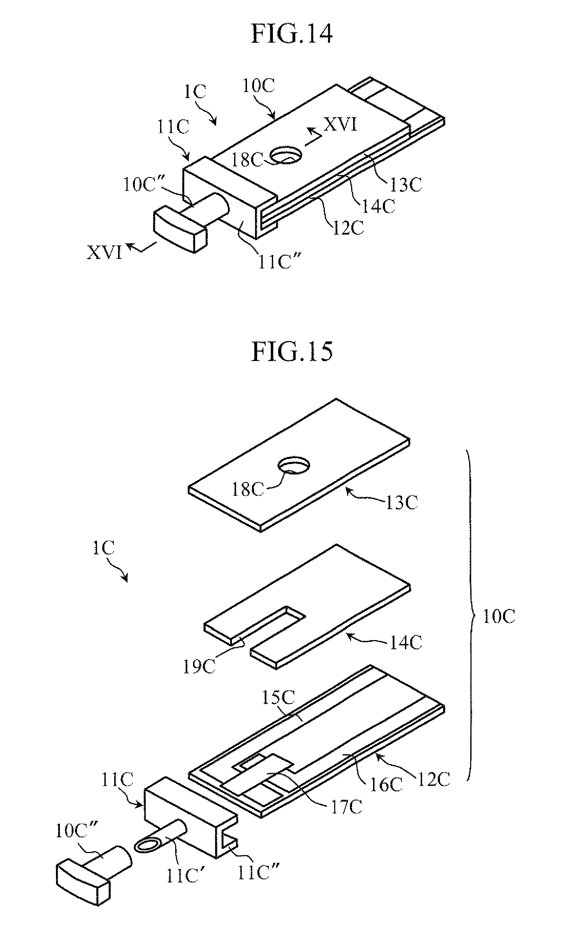

A fourth embodiment of the present invention will be described with reference to Fig. 14 to Fig. 16.

A sensor/lancet integrated device 1C shown in Fig. 14 to Fig. 16 includes a sensor 10C and a lancet 11C.

The sensor 10C is similar to the sensor 10B (see Fig. 11 to Fig. 13) according to the third embodiment described above. The sensor 10C has a spacer 14C interposed between a substrate 12C and a cover 13C, and includes a capillary 10C'. That is, the substrate 12C is formed with a pair of electrodes 15C, 16C and a reagent part 17C, a cover 13C includes a pass-through hole 18C, and the spacer 14C includes a slit 19C.

The lancet 11C includes a needle part 11C' and a joint 11C".

The needle part 11C' functions similar to the needle part 14 (see Fig. 1 and Fig. 2) of the previously described sensor/lancet integrated device 1, and is formed to a hollow form. The needle part 11C' is made from resin or metal, and is configured to act the capillary force. The interior of the needle part 11C' is communicated to the capillary 10C' of the sensor 10C. The needle part 11C' is insert molded with respect to the joint 11C".

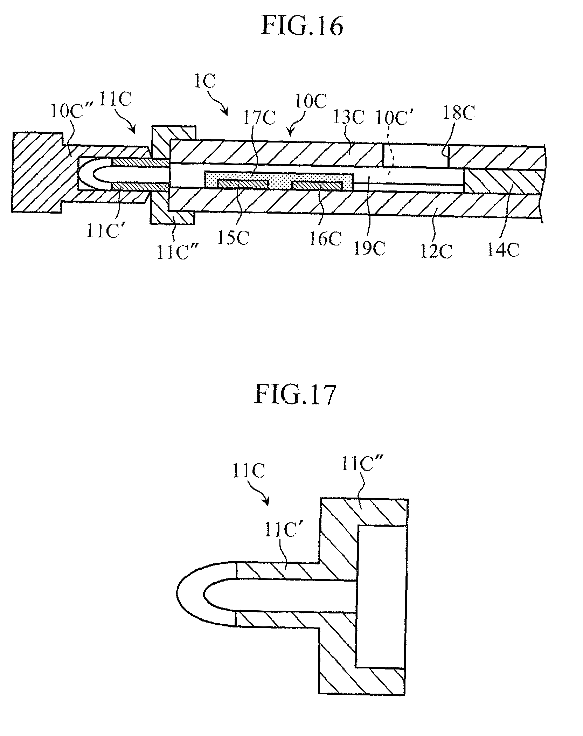

As shown in Fig. 14 and Fig. 16, the needle part 11C is covered by a cap 10C" similar to the sensor 10B (see Fig. 11 to Fig. 13) according to the previously described third embodiment. The cap 10C" may be integrally formed with the joint 11C" or may be formed as a separate body from the joint 11C". The contamination of the needle part 11C is prevented and the sterilization process of the needle part 11C' is appropriately maintained when the device 1C is not in use by arranging the cap 11C". The user will not be injured by the needle part 11C', or the user will not be contaminated by the blood attached to the needle part 11C' by attaching the cap 10C" to the needle part 11C' after using the device 1C.

The joint 11C" is provided to fix the lancet 11C to the sensor 10C. The joint 11C" sandwiches a substrate 12C, a cover 13C and the spacer 14C of the sensor 10C.

As shown in Fig. 17, the lancet 11C may have the needle part 11C' and the joint 11C" integrally formed by resin molding and the like.

The sensor/lancet integrated device 1C is used by being attached to the lancing device 2 described with reference to Fig. 3 and Fig. 4, similar to the previously described sensor/lancet integrated device 1 (see Fig. 1 and Fig. 2). In other words, in the sensor/lancet integrated device 1C, the device 1C is moved by the movement mechanism 21 (see Fig. 3 and Fig. 4) in the lancing device 2 to puncture the needle part 11C' to the skin and collect blood from the skin. As a method of collecting blood from the skin, the method described with reference to Fig. 6 or the method described with reference to Fig. 7 can be applied. The specific component in the blood can be analyzed by applying voltage between the electrodes 15C, 16C of the device 1C by the connector 22 (see Fig. 3 and Fig. 4), and measuring the response current value.

The sensor/lancet integrated device 1C has both the function of the sensor and the function of the lancet although the sensor 10C and the lancet 11C (needle part 11C') are formed as separate bodies by fixing the lancet 11C to the sensor 10C. The sensor/lancet integrated device 1C thus has small number of components and a simple configuration, and can be manufactured easily and effectively in terms of cost.

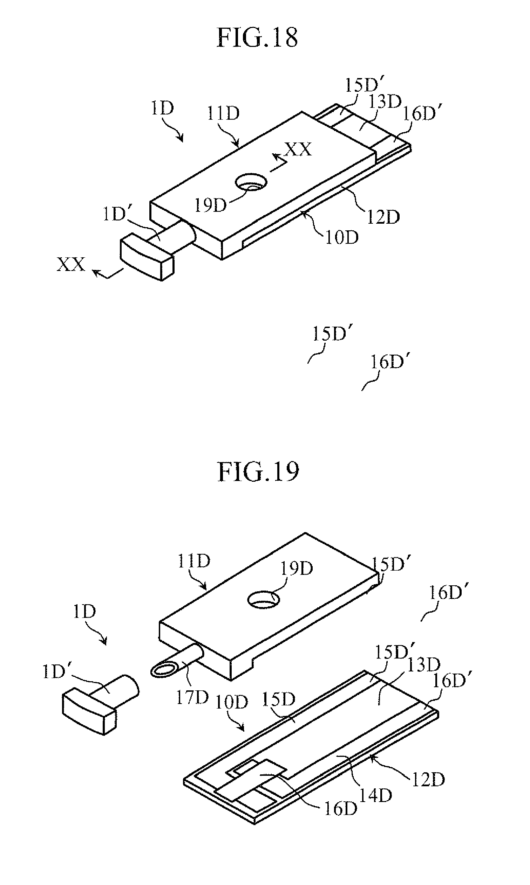

A fifth embodiment of the present invention will be described with reference to Fig. 18 to Fig. 20.

A sensor/lancet integrated device 1D shown in Fig. 18 to Fig. 20 includes a sensor 10D and a lancet 11D.

The sensor 10D has electrodes 14D, 15D and a reagent part 16D formed on an upper surface 13D of a substrate 12D.

The lancet 11D covers the sensor 10D while exposing ends 14D', 15D' of the electrodes 14D, 15D, and includes a needle part 17D, a groove 18D, and a pass-through hole 19D. The lancet 11D is joined to the sensor 10D through ultrasonic fusion bonding etc. The lancet 10D may be joined to the sensor 10D using a double-sided tape or an adhesive.

The needle part 17D functions similar to the needle part 14 (see Fig. 1 and Fig. 2) of the previously described sensor/lancet integrated device 1, and is formed to a hollow form so that capillary force acts. The needle part 11C is made from resin or metal. The needle part 17D is covered by a cap 1D'. The cap 1D' is, for example, integrally formed with respect to the lancet 11D. The interior of the needle part 17D is filled with resin. The resin is integrated to the cap 1D', and is removed with the cap 1D' by detaching the cap 1D'. Thus, the sterile state of the needle part 17D can be further maintained by filling the interior of the needle part 17D with resin.

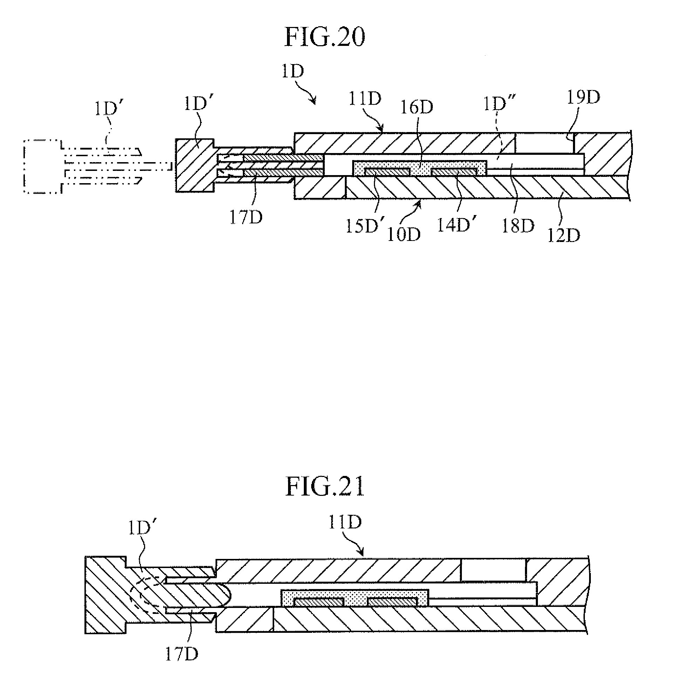

As shown in Fig. 21, the needle part 17D may be integrally formed to the lancet 11D. In this case, the cap 1D' is formed in a separate process from the lancet 11D. In the needle part 17D shown in Fig. 21 as well, the interior of the needle 17D is preferably filled with resin. The sterile state of the needle part 17D thus can be further maintained.

As shown in Fig. 20, the groove 18D defines a capillary 1D" when the lancet 11D is joined to the sensor 10D. The capillary 1D" is a space for acting the capillary force, and is communicated to the interior of the needle part 17D and the pass-through hole 19D.

The pass-through hole 19D discharges the gas of the interior of the needle part 17D and the interior of the capillary 1D".

The sensor/lancet integrated device 1D is used by being attached to the lancing device 2 described with reference to Fig. 3 and Fig. 4, similar to the previously described sensor/lancet integrated device 1 (see Fig. 1 and Fig. 2). In other words, in the sensor/lancet integrated device 1D, the device 1D is moved by the movement mechanism 21 (see Fig. 3 and Fig. 4) in the lancing device 2 to puncture the needle part 17D to the skin and collect blood from the skin. As a method of collecting blood from the skin, the method described with reference to Fig. 6 or the method described with reference to Fig. 7 can be applied. The specific component in the blood can be analyzed by applying voltage between the electrodes 14D, 15D of device 1D by the connector 22 (see Fig. 3 and Fig. 4), and measuring the response current value.

The sensor/lancet integrated device 1D has both the function of the sensor and the function of the lancet by joining the lancet 11D including the needle part 17D to the sensor 10D. The sensor/lancet integrated device 1D thus has small number of components and a simple configuration, can be manufactured easily and effectively in terms of cost.

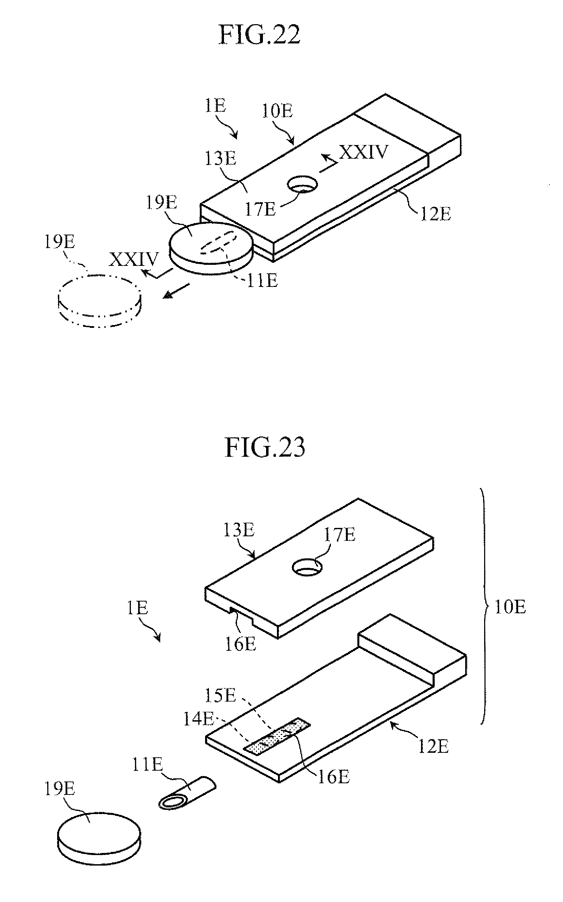

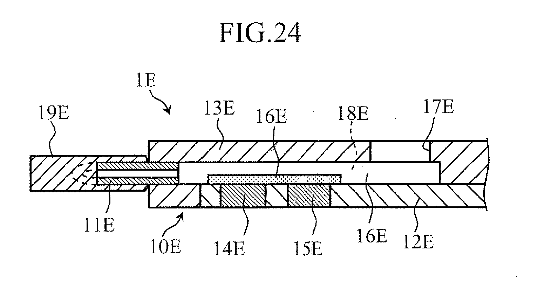

A sixth embodiment of the present invention will be described with reference to Fig. 22 to Fig. 24.

A sensor/lancet integrated device 1E shown in Fig. 22 to Fig. 24 includes a sensor 10E and a needle part 11E.

The sensor 10E has a cover 13E joined with respect to a substrate 12E, and is entirely formed to a plate shape.

A pair of electrodes 14E, 15E and a reagent part 16E are arranged on the substrate 12E. The pair of electrodes 14E, 15E, the pair of electrodes 15B, 16B functions similar to the pair of electrodes 11, 12 (see Fig. 1 and Fig. 2) of the previously described sensor/lancet integrated device 1, and is embedded in the substrate 12E while having both surfaces exposed. The reagent part 16E is formed bridging across the electrodes 14E, 15E.

The cover 13E covers the substrate 12E, and is joined to the substrate 12E through ultrasonic fusion bonding etc. The cover 13E may be joined to the substrate 12E using a double-sided tape or an adhesive. The cover 13E has a groove 16E and a pass-through hole 17E, where a capillary 18E is formed when the cover 13E is joined to the substrate 12E. The capillary 18E is a space for acting the capillary force, and is communicated to the interior of the needle part 11E.

The needle part 11E functions similar to the needle part 14 (see Fig. 1 and Fig. 2) of the previously described sensor/lancet integrated device 1, and is formed to a hollow form so that capillary force acts. The needle part 11C is made from resin or metal, and is fixed between the substrate 12E and the cover 13E at the entrance of the capillary 18E. The fixation of the needle part 11B can be carried out by sandwiching the needle part 11B between the substrate 12B and the cover 13B, or may be carried out using adhesive etc. The needle part 11B is covered by a cap 19E. The needle part 11E is also covered by the cap 19E, so that the needle part 11E is prevented from being contaminated and the user is prevented from being injured.

The sensor/lancet integrated device 1E is used by being attached to the lancing device 2 described with reference to Fig. 3 and Fig. 4, similar to the previously described sensor/lancet integrated device 1 (see Fig. 1 and Fig. 2). In other words, in the sensor/lancet integrated device 1B, the device 1B is moved by the movement mechanism 21 (see Fig. 3 and Fig. 4) in the lancing device 2 to puncture the needle part 11E to the skin and collect blood from the skin. As a method of collecting blood from the skin, the method described with reference to Fig. 6 or the method described with reference to Fig. 7 can be applied. The connector of the lancing device is design changed as necessary since the configuration of the electrodes 14E, 15E of the device 1E is different from those of the device 1 (see Fig. 1 and Fig. 2).

The sensor/lancet integrated device 1E has the needle part 11E formed as a separate body by resin molding etc., but has both the function of the sensor and the function of the lancet by fixing the needle part 11E at the entrance of the capillary 18E. The sensor/lancet integrated device 1E thus has small number of components and a simple configuration, can be manufactured easily and effectively in terms of cost.

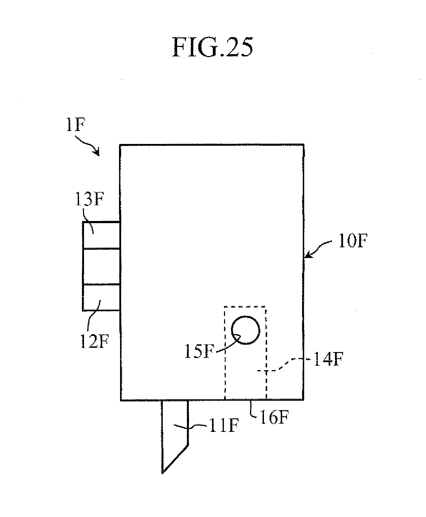

A seventh embodiment of the present invention will be described with reference to Fig. 25.

A sensor/lancet integrated device 1F shown in Fig. 25 has functions of both the sensor and the lancet, and includes a sensor part 10F and a needle part 11F.

The sensor part 10F includes terminals 12F, 13F, a capillary 14F, and an exhaust port 15F. The terminals 12F, 13F contact an external connector (not shown), and configure one part of the electrode. The capillary 14F aspirates blood by the capillary force and holds the blood, and includes an aspirating port 16F for introducing blood. The exhaust port 15F is provided to exhaust gas inside the capillary 14F when aspirating blood into the capillary 14F.

The needle part 11F is inserted to the skin when incising the skin, and is integrally molded with respect to the component of the sensor part 10F at the site adjacent to the aspirating port 16F. When forming the component of the sensor part 10F by resin molding, the needle part 11F is simultaneously integrally molded with such component or is integrated to the component of the sensor part 10F by insert molding the needle formed as a separate body.

The sensor/lancet integrated device 1F is used by being attached to the lancing device 2 described with reference to Fig. 3 and Fig. 4, similar to the previously described sensor/lancet integrated device 1 (see Fig. 1 and Fig. 2). In other words, in the sensor/lancet integrated device 1F, the device 1F is moved by the movement mechanism 21 (see Fig. 3 and Fig. 4) in the lancing device 2 to puncture the needle part 11F to the skin and collect blood from the skin. As a method of collecting blood from the skin, a method of bleeding blood from the incised site by generating negative pressure at the interior of the housing 20 (see Fig. 3 and Fig. 4) of the lancing device 2, for example, with the needle part 11F remaining inserted in the skin can be adopted. In this case, the aspirating port 16F of the capillary 14F is shifted from the needle part 11F, but the blood can be appropriately introduced from the aspirating port 16F when removing the needle part 11F from the skin by bringing the needle part 11F close to the aspirating port 16F. However, in the lancing device 2, the arrangement of the terminals of the connector is design changed such that voltage is appropriately applied to the electrodes (terminals 12F, 13F) of the device 1F at the connector 22.

In the sensor/lancet device 1F, the number of components is small, the configuration is simple, and the manufacturing is simplified since the needle part 11F is integrated to the component of the sensor part 10F. The function of the sensor and the function of the lancet can be provided to one device at low manufacturing cost.

In the sensor/lancet integrated device 1F, the terminals 12F, 13F do not need to be formed at the positions shown in Fig. 25 and may be formed at other locations, and the sensor part 11F is not limited to a rectangular shape in plan view and may be circular etc.

The sensor/lancet integrated device 1F may have a configuration in which the sensor part and the lancet part are integrated after forming the sensor part including a reagent part (capillary 14f) and the lancet part including the needle part 10F as separate bodies, for example, the sensor part may be accommodated in the lancet part as in the sensor/lancet integrated device 1A shown in Fig. 8 to Fig. 10.

The sensor/lancet integrated device 1F may also be configured as a colorimetric sensor by omitting the electrodes (terminals 12F, 13F), as well as containing color producing agent in the reagent part (not shown) and forming at least one part of the sensor part 10F to be transparent so that photometry of the reagent part is possible. The colorimetric sensor in this case may be configured to perform photometry of the reagent part based on transmitted light, or may be configured to perform photometry of the reagent part based on reflected light.

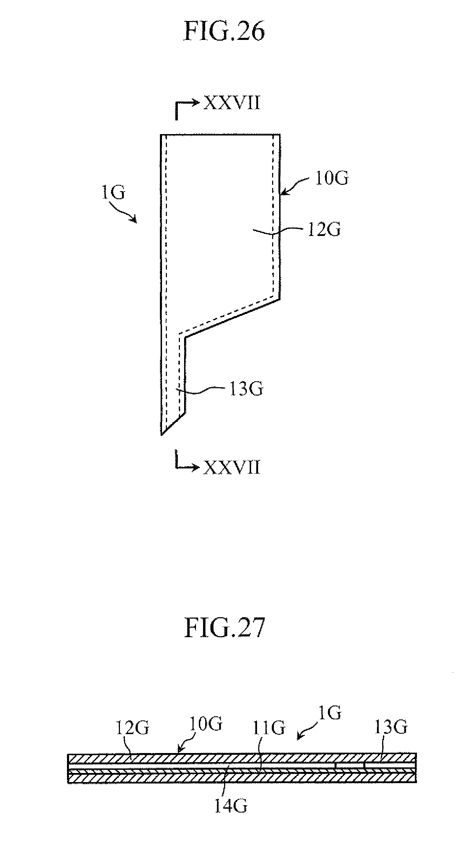

An eighth embodiment of the present invention will be described with reference to Fig. 26 and Fig. 27.

A sensor/lancet integrated device 1G shown in Fig. 26 and Fig. 27 has functions of both the sensor and the lancet, and is configured to analyze a specific component in the blood by colorimetry. The sensor/lancet integrated device 1G is substantially the sensor/lancet integrated device 1 according to the first embodiment omitted with the pair of electrodes 11, 12 (see Fig. 1 and Fig. 2), and includes a resin molded body 10G and a reagent part 11G.

The resin molded body 10G has a body part 12G and a needle part 13G, which are integrally formed by resin molding. The body part 12G has an internal space 14G for arranging a reagent part 11G. The internal space 14G is configured to act capillary force. The needle part 13G is formed to a hollow form, and is configured to act capillary force. The resin molded body 10G is formed transparent so that the color of the color producing agent in the reagent part 11G can be detected. In this case, the sensor/lancet integrated device 1G may be configured to detect the color of the color producing agent based on the transmitted light, or to detect based on the reflected light.

The reagent part 11G is arranged in the internal space 14G of the resin molded body 10G, and dissolves by the blood supplied to the internal space 14G. The reagent part 11G contains a color producing agent, and preferably further includes oxidation-reduction enzyme and electron transfer substance.

The sensor/lancet integrated device 1G is used by being attached to a lancing device equipped with a movement mechanism for moving the device 1G, and a photometric mechanism for measuring the extent of the color of the color producing agent of the reagent part 31. In other words, the sensor/lancet integrated device 1G that may be used has a photometric mechanism (e.g., photosensor) arranged in place of the terminal 29 at the connector in the lancing device 2 described with reference to Fig. 3 and Fig. 4. In this case, the method described with reference to Fig. 6, or the method described with reference to Fig. 7 can be applied.

The sensor/lancet integrated device 1G is substantially similar to the sensor/lancet integrated device 1 according to the first embodiment described with reference to Fig. 1 and Fig. 2 other than that the pair of electrodes 11, 12 are omitted, and thus effects similar to the sensor/lancet integrated device 1 can be obtained.

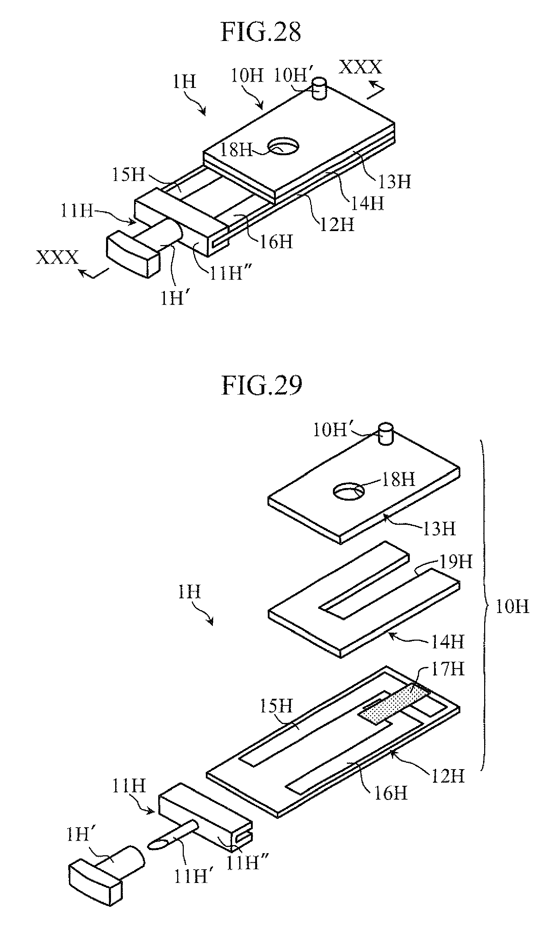

A ninth embodiment of the present invention will be described with reference to Fig. 28 to Fig. 36.

A sensor/lancet integrated device 1H shown in Fig. 28 to Fig. 30 has functions of both the sensor and the lancet, and includes a sensor 10H and a lancet 11H.

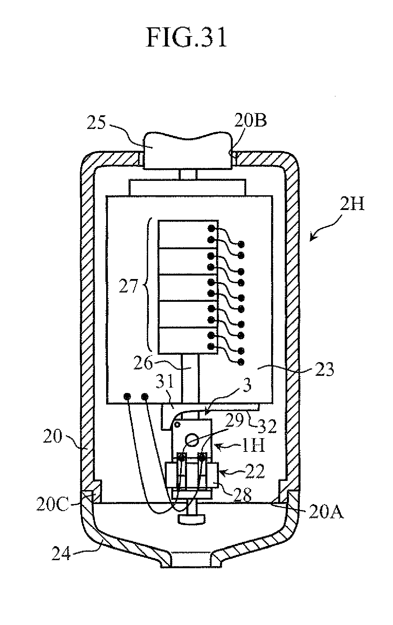

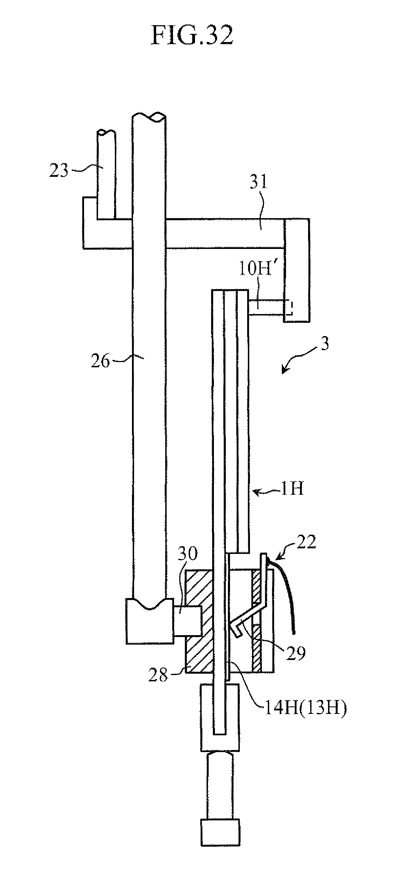

The sensor 10H is similar to the sensor 10C of the sensor/lancet integrated device 1C according to the fourth embodiment of the present invention described with reference to Fig. 14 to Fig. 16. That is, the sensor 10H has a spacer 14H interposed between a substrate 12H and a cover 13H, and includes a capillary 10H'. The substrate 12H is formed with a pair of electrodes 15H, 16H and a reagent part 17H, a cover 13H includes a pass-through hole 18H, and the spacer 14H includes a slit 19H. However, the sensor 10H has a projection 10H' arranged on the cover 13H. The projection 10H' is used to rotate the device 1H when using the device 1H by attaching to a lancing device 2H (Fig. 31 and Fig. 32) to be hereinafter described.

The lancet 11H includes a needle part 11H' and a joint 11H".

The needle part 11H' incises the skin, and is made of resin or metal. The needle part 11H' is fixed to the sensor 10H by way of the joint 11H". The needle part 11H' is integrally molded or is insert molded with respect to the joint 11H". The needle part 11H' is covered by a cap 1H', so that the needle part 11H' is prevented from being contaminated, and the user is prevented from being injured.

The joint 11H" is provided to fix the lancet 11H to the sensor 10H. The joint 11H" is fixed at the end on the opposite side of an introduction port of the capillary 10H' in the substrate 12H. That is, the needle part 11H' is not communicated to the capillary 10H', and is projected out from the opposite side of the introduction port of the capillary 10H'. In such configuration, the joint 11H" can be fixed to the sensor 10H after sterilizing the joint 11H" separate from the sensor 10H. Thus, the reagent part of the sensor 11H will not degrade when sterilizing the joint 11H" (needle part 11H').

As shown in Fig. 31 and Fig. 32, the sensor/lancet integrated device 1H is used by being attached to the lancing device 2H. The basic configuration of the lancing device 2H is similar to the lancing device 2 (see Fig. 3 and Fig. 4) according to the first embodiment, but differs in that a rotation mechanism 3 is arranged. In the drawings referenced below, same reference numerals are denoted for the elements similar to the lancing device 2 (see Fig. 3 and Fig. 4), and redundant description will be omitted.

The rotation mechanism 3 is provided to rotate the device 1H attached to the connector 22. The rotation mechanism 3 includes a rotation shaft 30 and a guide 31.

The rotation shaft 30 is rotatably coupled to the lower end of the rod 26 and is non-rotatably coupled to the connector 22. That is, the rotation shaft 30 is rotatable with respect to the rod 26 along with the connector 22. The rotation shaft 30 is non-rotatably coupled to the connector 22, and the rotation shaft 30 is rotatably coupled to the rod 26, so that the connector 22 is rotatable with respect to the rod 26.

The guide 31 regulates the rotary operation of the device 1H, and is the portion to which the projection 10H' of the device 1H engages. The guide 31 has a curved surface 32 for engaging the projection 10H', so that the projection 10H' slidably moves on the curved surface 32 when the device 1H moves upward with the connector 22.

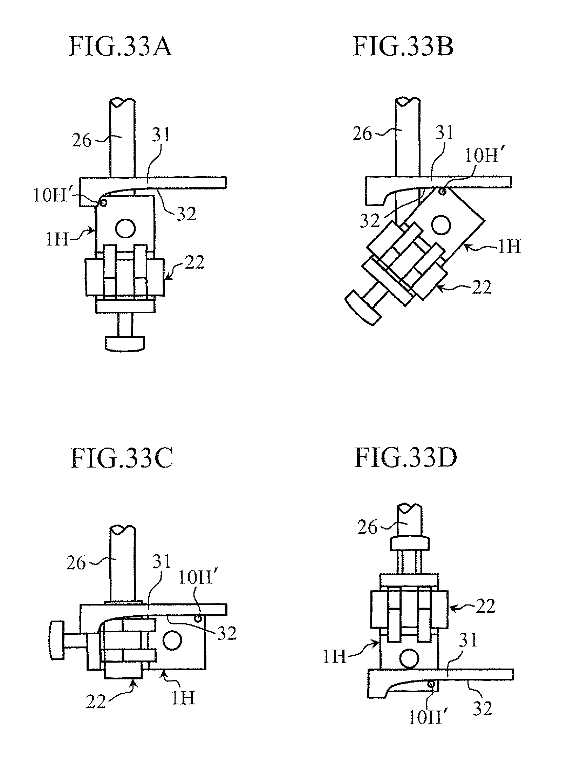

More specifically, as shown in Fig. 33A, the projection 10H' of the device 1H interferes with the curved surface 32 of the guide 31 when the device 1H is attached to the connector 22 and the device 1H is moved upward with the connector 22. As shown in Fig. 32B to Fig. 32D, the projection 10H' slidably moves on the curved surface 32 of the guide 31, and the device 1H is rotated by 180 degrees with the connector 22 when the connector 22 and the device 1H are moved upward.

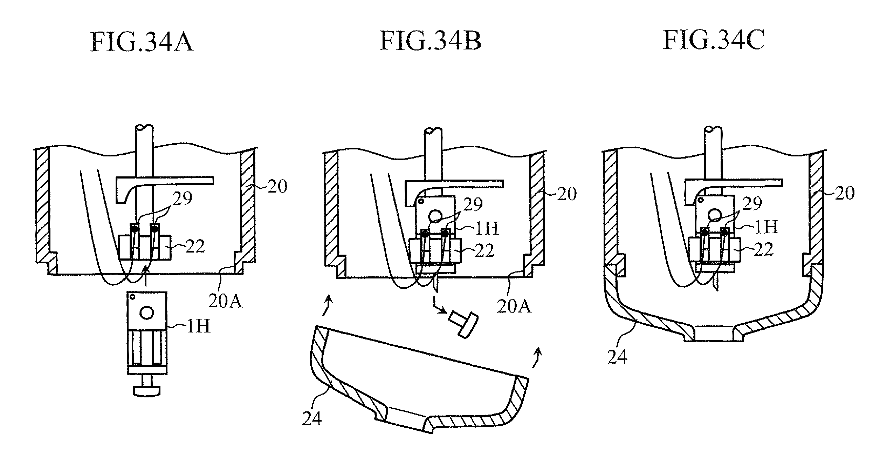

The measuring operation using the device 1 and the lancing device 2H will be described with reference to Fig. 34 to Fig. 36.

As shown in Fig. 34A to Fig. 34C, the cap 24 is detached from the housing 20 and the opening 20A is opened. The device 1H is inserted to the connector 22 in this state. When the device 1H is inserted to the connector 22, the terminal 29 of the connector 22 contacts the electrodes 15H, 16H (see Fig. 32) of the device 1H, and the terminal 29 acts a pushing force on the device 1H. The device 1H is thereby held by the connector 22. After the attachment of the device 1H, the cap 1H' is detached from the device 1H and the opening 20A is covered by the cap 24.

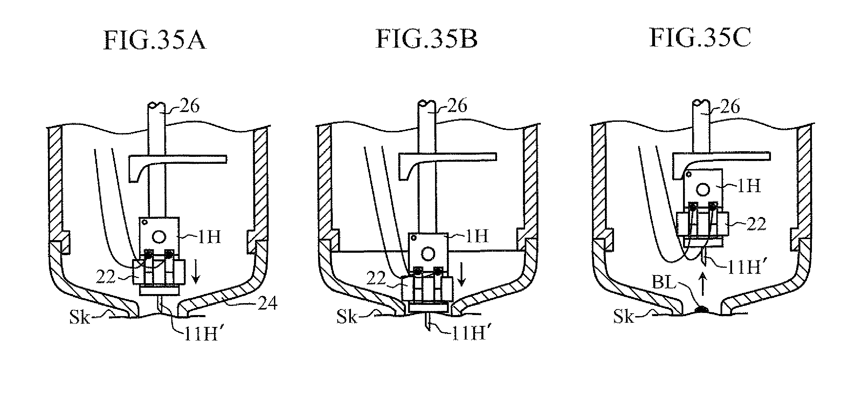

As shown in Fig. 35A, the operation button 25 is pushed with the distal end of the cap 24 contacting the skin Sk. A measurement start signal is thereby generated, and such signal is provided to the control plate 23. When detecting such signal, the control plate 23 controls the actuator 27 and moves the rod 26 downward. The connector 22 and the device 1H are then moved downward with the rod 26. When the tip of the needle part 11H of the device 1H is contacted to the skin Sk, the control plate 23 obtains the movement distance of the rod 26 to this point. That is, the control plate 23 obtains the distance to the skin Sk from the state shown in Fig. 34C. Whether or not the tip of the needle part 11H' in the device 1H has contacted the skin is determined by detecting the time point at which the load acting on the rod 26 becomes large by a pressure sensor, a position sensor, and the like.

As shown in Fig. 35B, the control plate 23 further moves the rod 26, and furthermore, the device 1H downward to puncture the skin Sk with the needle part 11H of the device 1H. Thereafter, as shown in Fig. 35C, the control plate 23 controls the actuator 27 after puncturing the skin Sk with the needle part 11H, moves the rod 26 and furthermore the device 1H upward to remove the needle part 11H from the skin Sk. In this case, the blood BL bleeds from the puncture site since the puncture site in the skin Sk is incised by the needle part 11H.

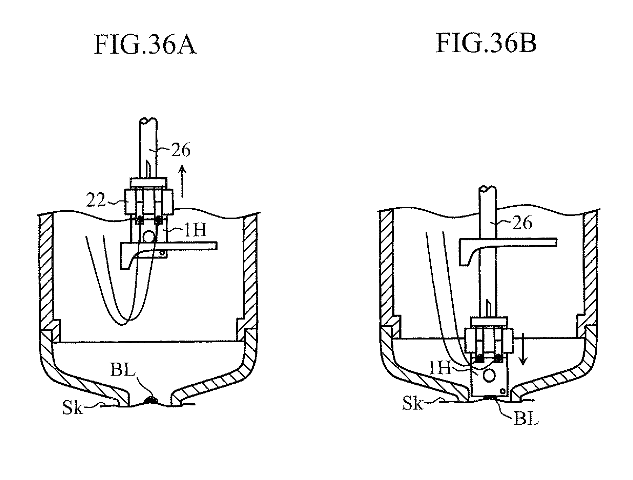

As shown in Fig. 36A, the control plate 23 controls the actuator 27 and further moves the rod 26 and furthermore the device 1H upward, and rotates the device 1H 180 degrees, as described with reference to Fig. 32A to Fig. 32D.

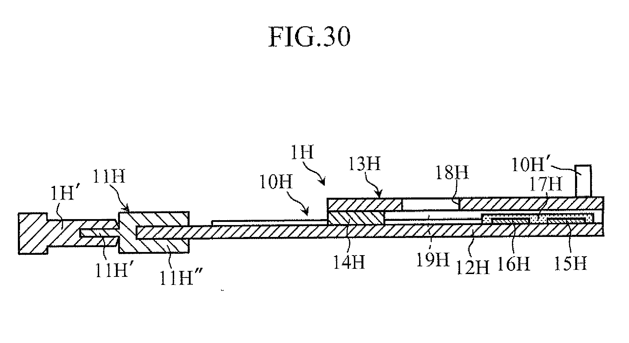

As shown in Fig. 36B, the control plate 23 controls the actuator 27 and moves the rod 26 and furthermore the device 1H downward, and contacts the introduction port of the capillary 19H (see Fig. 30) to the skin Sk. The needle part 11H' of the device 1H is appropriately contacted to the skin Sk since the distance to the skin Sk is known in the operation shown in Fig. 35A. As the blood BL is bleeding from the puncture site from the skin Sk, the tip of the needle part 11H' is contacted to the blood BL. The needle part 11H' is formed such that the capillary force can act as described above, the blood BL bleeding from the skin Sk is introduced to the capillary 19H of the device 1H shown in Fig. 30 through the needle part 11H'. The capillary 19H is filled with blood BL since the capillary 19H is also formed to act the capillary force. The reagent part 17H is dissolved, so that a reaction system containing the reagent and the blood is formed inside the capillary 19H.

As apparent with reference to Fig. 30 to Fig. 32, the control plate 23 applies voltage between the terminals 29 when detecting a signal generated by the pushing of the operation button 25. The voltage is thus applied to the reaction system of the capillary 19H. The response current is measured by the terminal 29. The control plate 23 also calculates the concentration of the specific component in the blood based on the response current measured by the terminal 29. Such calculation can be performed based on a known method such as an analytical curve showing a relationship between the response current value and the concentration.

In the sensor/lancet integrated device 1H of the present embodiment, the needle part 11H' is integrated with the joint 11H" by integrally molding or insert molding the lancet 11H. The sensor/lancet integrated device 1H has small number of components, the configuration is simple, and a function of the sensor and a function of a lancet can be provided to one device at low cost. Since the device 1H can be manufactured at low cost, the blood collecting cost can be reduced in the blood collecting method using the device 1H.

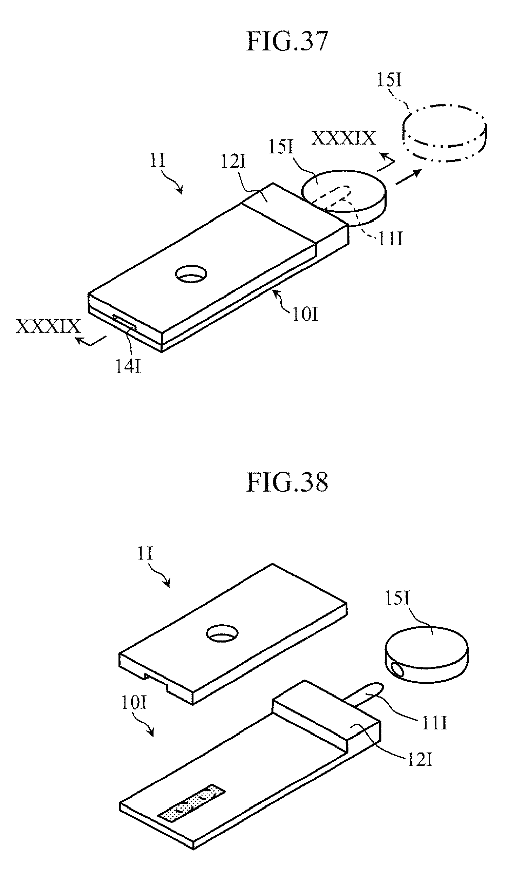

A tenth embodiment of the present invention will now be described with reference to Fig. 37 to Fig. 39.

A sensor/lancet integrated device 11 shown in Fig. 37 to Fig. 39 includes a sensor 101 and a needle part 11I, and the basic configuration thereof is similar to the device 1E described with reference to Fig. 22 to Fig. 24.

The needle part 11I projects to the side opposite to an aspirating port 14I of a capillary 13I at a bulging-out part 121 of the sensor 10I. The needle part 11I is integrally molded at the bulging-out part 121 of the sensor 10I by resin molding, or is insert molded with respect to the bulging-out part 12I. The needle part 11I is covered by a cap 15I, and the needle part 11I is prevented from being contaminated and the user is prevented from being injured.

The device 1I is used by being attached to the lancing device 2H described with reference to Fig. 31 and Fig. 32 since the aspirating port 14I and the needle part 11I are positioned opposite to each other. That is, after puncturing the skin with the needle part 11I, the device 1I is rotated by 180 degrees, and the aspirating port 14I is contacted to the bleeding site of the skin to introduce blood into the capillary 13I.

In the sensor/lancet integrated device 1I of the present embodiment, the needle part 11I is integrated with the sensor 10I by resin molding or by insert molding. The sensor/lancet integrated device 1I thus has small number of components, the configuration is simple, and a function of the sensor and a function of a lancet can be provided to one device at low cost. Since the device 1I can be manufactured at low cost, the blood collecting cost can be reduced in the blood collecting method using the device 1I.

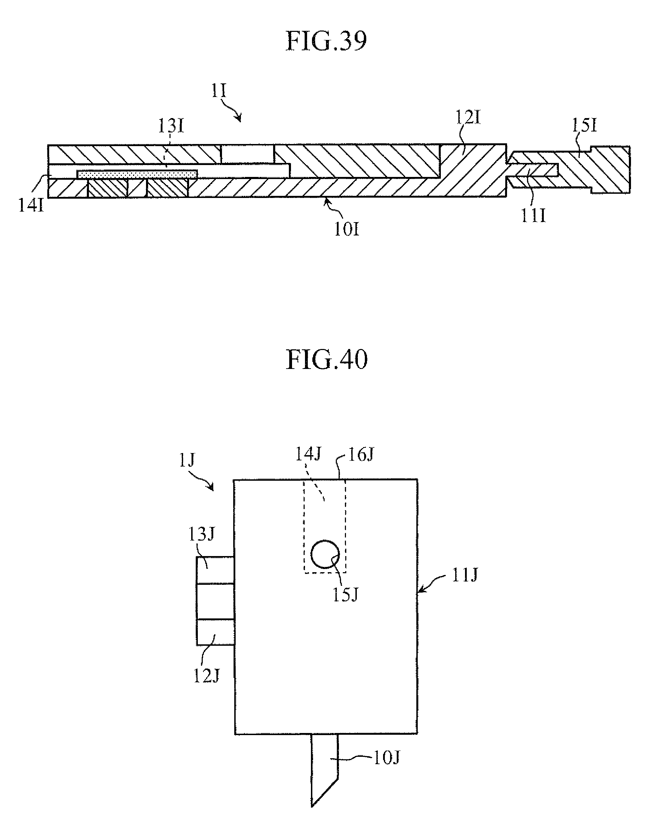

An eleventh embodiment of the present invention will be described with reference to Fig. 40.

A sensor/lancet integrated device 1J shown in Fig. 40 has functions of both a sensor and a lancet, and includes a sensor part 10J and a needle part 11J. The basic configuration of the device 1J is similar to the device 1F described with reference to Fig. 25.

The device 1J has the needle part 11J formed on the side opposite to an aspirating port 13J of the capillary 12J, different from the device 1F (see Fig. 25). When forming the component of the sensor part 10J by resin molding, the needle part 11J is simultaneously integrally molded with the component or the needle formed as a separate body is integrated with the component of the sensor part 10J by insert molding.

The device IJ is used by being attached to the lancing device 2H described with reference to Fig. 31 and Fig. 32 since the aspirating port 13J and the needle part 11J are positioned opposite to each other. That is, after puncturing the skin with the needle part 11J, the device 1J is rotated by 180 degrees, and the aspirating port 13J is contacted to the bleeding site of the skin to introduce blood into the capillary 12J. However, in the lancing device, the arrangement of the terminal of the connector is design changed such that voltage can be appropriately applied to the electrodes (terminals 14J, 15J) of the device 1J at the connector.

In the sensor/lancet integrated device 1J of the present embodiment, the needle part 11J is integrated with the sensor part 10J by resin molding or by insert molding. The sensor/lancet integrated device 1J thus has small number of components, the configuration is simple, and a function of the sensor and a function of a lancet can be provided to one device at low cost. Since the device 1J can be manufactured at low cost, the blood collecting cost can be reduced in the blood collecting method using the device 1J.

In the sensor/lancet integrated device 1J, the terminals 14J, 15J do not need to be formed at the illustrated positions and may be formed at other locations, and the sensor part 11J is not limited to a rectangular shape in plan view and may be circular etc.

The sensor/lancet integrated device 1J may have a configuration in which the sensor part and the lancet part are integrated after forming the sensor part including a reagent part (capillary 16J) and the lancet part including the needle part 11J as separate bodies, for example, the sensor part may be accommodated in the lancet part as in the sensor/lancet integrated device 1A shown in Fig. 8 to Fig. 10.

The sensor/lancet integrated device 1J may also be configured as a colorimetric sensor by omitting the electrodes (terminals 14J, 15J), as well as containing a color producing agent in the reagent part (not shown) and forming at least one part of the sensor part 10J to be transparent so that photometry of the reagent part is possible. The colorimetric sensor in this case may be configured to perform photometry of the reagent part based on transmitted light, or may be configured to perform photometry of the reagent part based on reflected light.

The rotation mechanism 3 of the lancing device 2H using the device 1H, 11, 1J is not limited to the configuration including the rotation shaft 30 and the guide 31, and may adopt other configurations. A rotation mechanism that uses the rotation force of a small motor may be used for the rotation mechanism. The rotation angle of the device by the rotation mechanism is determined by the position relationship of the needle part and the aspirating port in the device, and the rotation angle by the rotation mechanism is set in a range of between 0 to 180 degrees according to such position relationship.

## Claims

1. A sensor/lancet integrated device comprising a lancet including a needle part for puncturing skin, and a sensor including a reagent part; wherein
the lancet is integrally molded with the needle part.

2. The sensor/lancet integrated device according to claim 1, wherein the sensor further includes an electrode integrally incorporated in the lancet.

3. The sensor/lancet integrated device according to claim 1, wherein
the sensor and the lancet are formed as separate bodies; and
the sensor is held in an internal space formed in the lancet to be integrated to each other.

4. The lancet/lancet integrated device according to claim 1, wherein
the sensor has a mode in which a cover is joined with respect to a substrate;
the lancet includes a joint for attaching to the sensor; and
the joint is fixed to the sensor so as to sandwich the substrate.

5. The lancet/lancet integrated device according to claim 4, wherein the joint is fixed to the sensor so as to further sandwich the cover.

6. The lancet/lancet integrated device according to claim 5, further comprising:
a capillary for supplying body fluid to the reagent part; wherein
the needle part is projected from a location different from an introduction port of the body fluid in the capillary.

7. The sensor/lancet integrated device according to claim 1, wherein
the reagent part includes a color producing agent and is formed in an internal space formed in the lancet; and
at least one part of the lancet is formed transparent to measure a color of the color producing agent.

8. The sensor/lancet integrated device according to claim 1, wherein the needle part is formed hollow.

9. The lancet/lancet integrated device according to claim 8, further comprising:
a capillary for supplying body fluid to the reagent part; wherein
the interior of the needle part is communicated to the interior of the capillary.

10. The sensor/lancet integrated device according to claim 8, wherein the interior of the needle part is filled with resin before use.

11. The lancet/lancet integrated device according to claim 9, further comprising:
a capillary for supplying body fluid to the reagent part; wherein
the lancet includes a groove for defining the capillary.

12. A sensor/lancet integrated device comprising a lancet including a needle part for puncturing skin, and a sensor including an introduction port for introducing body fluid; wherein
the needle part is formed projecting from a location different from the introduction port.

13. The sensor/lancet integrated device according to claim 12, wherein the needle part is integrally molded to a component of the sensor.

14. The sensor/lancet integrated device according to claim 12, further comprising:
a cover to be joined to the sensor; wherein
the cover includes a groove for defining a capillary for introducing the body fluid.

15. The sensor/lancet integrated device according to claim 12, wherein the sensor further includes an electrode.

16. The sensor/lancet integrated device according to claim 12, wherein
the sensor and the lancet are formed as separate bodies; and
the sensor is held in an internal space formed in the lancet to be integrated to each other.

17. The sensor/lancet integrated device according to claim 12, wherein
the reagent part includes a color producing agent and is formed in an internal space formed in the lancet; and
at least one part of the lancet is formed transparent to measure a color of the color producing agent.

18. A sensor-run set integrated device comprising a lancet including a needle part for puncturing skin, and a sensor including a reagent part; wherein
the sensor has a mode in which a cover is joined with respect to a substrate; and
the needle part is fixed between the substrate and the cover.

19. A sensor/lancet integrated device comprising a lancet including a needle part for puncturing skin, and a sensor including a reagent part; wherein
the lancet includes a joint for fixing to the sensor; and
the needle part is integrated to the joint.

20. The lancet/lancet integrated device according to claim 19, wherein
the sensor has a mode in which a cover is joined with respect to a substrate; and
the joint is attached to the sensor so as to sandwich the substrate.

21. The lancet/lancet integrated device according to claim 20, wherein the joint is fixed to the sensor so as to further sandwich the cover.

22. A body fluid collecting method using a sensor/lancet integrated device including a lancet integrally molded with a needle part formed to a hollow form, and a sensor with a reagent part; the method comprising:
a first step of moving the needle part to a position where a tip of the needle part contacts skin, and obtaining the position where the tip of the needle part contacts the skin;
a second step of puncturing the needle part to the skin and incising the skin;
a third step of removing the needle part from the skin; and
a fourth step of contacting the tip of the needle part to the body fluid bled from the skin based on a result in the first step.

23. The body fluid collecting method according to claim 22, wherein the fourth step is performed by again moving the needle part after once being moved to a position where the tip of the needle part is completely away from the skin, and contacting the needle part to the body fluid bled from the skin.

24. The body fluid collecting method according to claim 22, wherein the fourth step is performed by stopping the movement of the needle part immediately after removing the needle part from the skin.

25. The body fluid collecting method according to claim 22, wherein at least one of the second step or the third step is performed with a negative pressure applied to the skin.

26. A body fluid collecting method using a sensor/lancet integrated device including a lancet integrally molded with a needle part formed to a hollow form, and a sensor with a reagent part; the method comprising:
a first step of puncturing the needle part to skin and incising the skin; and
a second step of aspirating the body fluid to an interior of the needle part by a capillary force generated in the interior of the needle part with the needle part punctured in the skin.

27. A body fluid collecting method using a sensor/lancet integrated device including a sensor with an introduction port for introducing body fluid, and a lancet with a needle part formed projecting from a location different from the introduction port; the method comprising:
a first step of puncturing the needle part to skin and incising the skin;
a second step of removing the needle part from the skin;
a third step of rotating the sensor/lancet integrated device; and
a fourth step of contacting the introduction port to the body fluid bled from the skin.

28. The body fluid collecting method according to claim 27, further comprising a fifth step, performed before the first step, of moving the needle part to a position where a tip of the needle part contacts the skin, and obtaining the position where the tip of the needle part contacts the skin.
